(19) Europäisches Patentamt
European Patent Office
Office européen des brevets

(11) Publication number: 0 347 724 A1

# (12) EUROPEAN PATENT APPLICATION

(21) Application number: 89110722.9

(22) Date of filing: 13.06.89

(51) Int. Cl.4: C07D 211/46 , C07D 207/16 , C07D 223/08 , C11D 3/395

(30) Priority: 14.06.88 IT 2095788

(43) Date of publication of application: 27.12.89 Bulletin 89/52

(84) Designated Contracting States: AT BE CH DE ES FR GB LI NL SE

(71) Applicant: AUSIMONT S.r.l.
31, Foro Buonaparte
I-20100 Milano(IT)

(72) Inventor: Venturello, Carlo, Dr. Chem.
135, via 23 Marzo
I-28100 Novara(IT)
Inventor: Cavallotti, Claudio
3, via Lorenteggio
I-20146 Milan(IT)

(74) Representative: Weinhold, Peter, Dr. et al
Patentanwälte Dipl.-Ing. G. Dannenberg Dr. P. Weinhold Dr. D. Gudel Dipl.-Ing. S. Schubert Dr. P. Barz Siegfriedstrasse 8
D-8000 München 40(DE)

## (54) Heterocyclic peracids having an amide function.

(57) Disclosed are heterocyclic (poly)percarboxylic acids with an amide function having the formula:

$R - (CH_2)_m - R'$ (I)

wherein:

R and R′, the same or different from each other, represent hydrogen or a group of the formula:

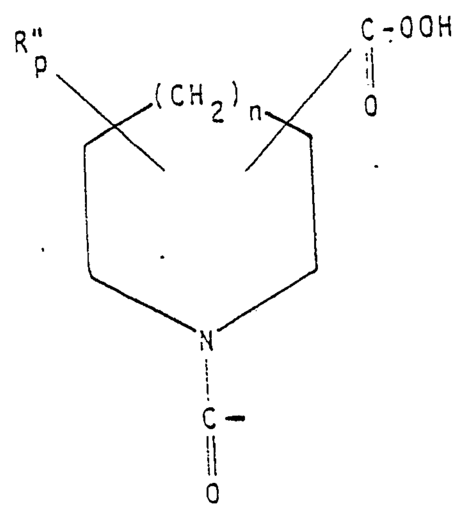

provided that at least one of R and R′ is different from H, and wherein

R″ represents a hydrogen atom or substituent which is inert towards the active oxygen of the percarboxylic group and/or the preparation conditions;

m represents an integer from 1 to 12;

n is selected from 0,1 and 2;

EP 0 347 724 A1

p is an integer of from 1 to 3.

Also described is a process for their preparation and their use as bleaching agents.

## HETEROCYCLIC PERACIDS HAVING AN AMIDE FUNCTION

The present invention relates to new derivatives of (poly)percarboxylic acids which can be referred to as heterocyclic (poly)percarboxylic acids having an amide group, a process for the preparation of said compounds and to their use in bleaching compositions.

In particular, the present invention relates to heterocyclic (poly)percarboxylic acids with at least one amide group having the formula (I):

$R - (CH_2)_m - R'$ (I)

wherein:

R and R′, the same or different from each other, represent hydrogen or a group of the following formula:

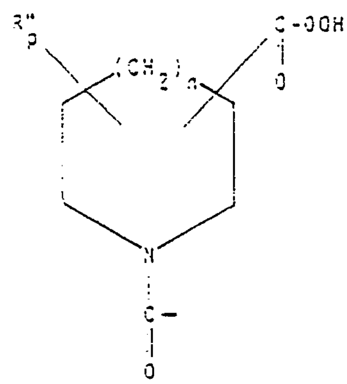

with the proviso that at least one of R and R′ is different from H, and wherein

R″ represents hydrogen or any other substituent which is inert (non-reactive) in the presence of the active oxygen of the percarboxylic acid group and/or under the preparation conditions;

m is an integer of from 1 to 12 inclusive;

n is selected from 0,1 and 2; and

p represents an integer of from 1 to 3 inclusive.

The heterocyclic percarboxylic compounds having the above formula (I) constitute a new class of products highly interesting from an industrial point of view.

In fact, they can find general use, similarly to already known peracids, e.g. in the field of plastics, as polymerization ini tiators and, in particular, as oxidants for olefin epoxidation and hydroxylation, and in many other oxidative processes in the field of fine chemistry.

Particularly interesting and efficient, however, is their application in the field of bleaching, i.e. in detergents.

In the past years, organic peracids have attracted increasing interest in the industrial field, due to their excellent properties when used as bleaching agents in compositions for medium/low temperature washing, and particularly in view of energy saving considerations.

Consequently. there is considerable research activity aiming at finding new organic peracid compounds having the necessary properties. i.e. high bleaching activity, thermal stability and storage stability or shelf life. The latter requirements are essential for an industrial application and a widespread use of such compounds.

Therefore, many mono- and di-percarboxylic acids, straight-chained or cyclic, are known and used, among others, in the field of detergence.

Already described percarboxylic acids are, e.g.: diperdodecanedioic acid, monoperphthalic acid, diperazelaic acid and substituted diperglutaric and-adipic acids and acylamino-(alkyl)arylen-percarboxylic acids. Said acids, however, are not heterocyclic percarboxylic acids.

One object of the present invention is to provide novel compounds, i.e. heterocyclic (poly)percarboxylic acids having an amide function and showing the above formula (I).

Another object of the present invention is to provide a simple and inexpensive process for the preparation of the above percarboxylic acids of formula (I).

A further object of the present invention is the use of the heterocyclic percarboxylic acids of formula (I) as bleaching agents in detergent formulations, particularly those for low-medium temperature use.

These, and still other objects, which will become evident from the following detailed disclosure, are

achieved, according to the present invention, by the nitrogen containing heterocyclic percarboxylic acids of the above formula (I), and by the process for the preparation thereof.

The present peracids may be obtained by essentially conventional methods. For example, they may be prepared by reacting the substrate, i.e. a heterocyclic (poly)carboxylic acid with amide function which corresponds in structure to the desired peracid of formula (I), with $H_2O_2$ in concentrated methanesulphonic acid and subsequent separation etc., according to known techniques.

In this way the percarboxylic acids of formula (I) may be obtained in the form of stable solids.

Stated more precisely, the process according to the present invention comprises the percarboxylation reaction with $H_2O_2$ of the substrate which corresponds to the desired acid of formula (I), in an acid medium, for example methanesulphonic acid.

The obtained product may then be filtered, extracted with a solvent (methylenechloride etc.), dried, etc., according to conventional techniques.

As stated above, the substrate used as starting material is the heterocyclic (poly)carboxylic acid corresponding to the desired (poly)percarboxylic acid of formula (I); these compounds are known, and/or can be prepared according to known methods.

Referring to the above formula (I), $R''$ preferably is hydrogen or a (linear or branched) alkyl, cycloalkyl, alkyl-aryl or aryl-alkyl group containing an overall number of up to 10, particularly up to 7, carbon atoms.

Said groups may in turn be substituted with one or more (preferably 1 to 3) atoms or groups, the same or different from each other and inert under the reaction conditions under which the preparation takes place such as, e.g., F, Cl, $NO_2$, $C_{1-5}$ alkoxy etc.

Alternatively, $R''$ represents any other substituent which does not react with the active oxygen of the percarboxylic group, e.g., a carboxylic group, a percarboxylic group, F, Cl, $NO_2$, $(C_{1-5})$-alkoxy etc.

Specific examples of the generic groups mentioned above are:

alkyl: methyl, ethyl, n- and i-propyl, n-, i-. sek.- and tert. butyl, pentyl, hexyl, heptyl, octyl, nonyl, decyl,
cycloalkyl: cyclopentyl, cyclohexyl, cycloheptyl and cyclooctyl;
alkylaryl: tolyl, xylyl, ethylphenyl and methylnaphthyl;
arylalkyl: benzyl, phenethyl and naphthylmethyl;
alkoxy: methoxy, ethoxy, propoxy, butoxy and pentoxy.

Preferably the index n is 1, whereas a preferred meaning of R(and $R''$) is hydrogen. If p is 2 or 3, the groups $R''$ may be the same or different from each other.

Examples of suitable substrates are N-acetyl-4-piperidine carboxylic acid,N-decanoyl 4-piperidine carboxylic acid, N-N′-adipoyl-bis (4-piperidine carboxylic acid), N-decanoyl-3-piperidinecarboxylic acid, N, N′-succinyl-bis (3-piperidine carboxylic acid), N, N′-glutaryl-bis (3-piperidine carboxylic acid), N-hexanoyl-4-piperidine carboxylic acid, N-decanoyl-6 methyl-4-piperidine carboxylic acid.

For $R''$ = COOH, preferably in a non-ortho-position with respect to the nitrogen atom, the peroxycarboxylation of $R''$ can be carried out, too, thus obtaining a product of formula (I) with two or more (preferably at the most four) percarboxylic groups.

In case of the presence of two adjacent carboxylic groups ($R''$ = COOH) the corresponding anhydride can be used, too.

According to a preferred mode of operation, using methanesulphonic acid, the percarboxylation reaction of the acid or poly-acid used as a starting material is carried out by gradually adding $H_2O_2$ having a concentration within the range of from about 70% to about 90% by weight, to a solution of the substrate in $CH_3SO_3H$, maintaining the reaction temperature throughout the reaction at at least 0 °C, preferably between about 10 and about 30 °C, dependent on the reactivity of the substrate.

The amount of $CH_3SO_3H$, determined at a concentration of 100%, preferably is at least 5 moles per each mole of substrate. Normally said amount ranges from 8 to 30 moles per each mole of substrate, and preferably is from about 10 to about 14 moles per mole of substrate.

The hydrogen peroxide is preferably used in an amount which is in an excess of at least 2 moles per each mole of substrate. Usually its amount ranges from 3 to 10 moles per mole of substrate, preferably of from 4 to 6 moles per mole of substrate, based on the COOH groups to be percarboxylated.

The reaction time depends on the nature of the substrate, the operating temperature, and the final $CH_3SO_3H/H_2O$ molar ratio present at the end of the reaction. Said ratio is preferably chosen to range from about 2 to about 6, preferably from about 3 to about 5, by adjusting the various parameters concerned.

Reaction times of from about 30 minutes to about 4 hours have proven to be suitable ones.

The separation of the heterocyclic (poly)percarboxylic acid of formula (I) may be carried out according to conventional methods, such as filtration of the solid precipitate, obtained after the treatment of the reaction mixture with an ammonium sulfate solution, or extraction with solvent etc.

The compounds of formula (I) are thus obtained as crystalline solids.

The compounds of formula (I) are usually solid at room temperature. According to the present invention they can be used in formulations of detergent compositions, e.g., granular formulations,and as bleaching agents in solution within a wide temperature range, for example between about 20° and about 90° C.

Therefore, the heterocyclic peracids of the present invention may directly be used as bleaching agents, separate from the detergent composition, but preferably are combined with and incorporated into the conventional detergent compositions which operate in the above temperature range and comprise other components and/or additives, such as, for example, builders, surfactants, soaps, zeolites, hydrotropic agents, corrosion inhibitors, enzymes, optical bleaching agents, stabilizers, other bleaching compounds, etc.

Preferably, the operating temperature lies between room temperature and about 65° C.

The preparation processes and the use in compositions as well as the formulations thereof are within the scope of the disclosed and/or conventional ones.

The peracids of formula (I) of the present invention may be used in combination with solid and liquid detergent compositions, and/or in the presence of other peroxidic bleaching compounds.

Finally, the heterocyclic peracids of the present invention are compatible with phlegmatization according to the conventional methods.

The present invention will now be described in more detail in the following examples, which are given for purely illustrative purposes.

The products prepared in the examples were characterized by elemental analysis, by determining their content of active oxygen (by iodometric titration), and by using Fourier Transform Infrared Spectroscopy (FT-IR).

## EXAMPLE 1

11 g of methanesulphonic acid (0.114 moles) and 2 g of N-acetyl4-piperidine carboxylic acid (0.0116 moles) were introduced into a beaker equipped with stirrer, thermometer and external bath. The mixture was stirred at 20 to 25° C until complete dissolution. The temperature was then lowered to 5° C and 2 g of $H_2O_2$ (70%, 0.0412 moles) were slowly introduced under stirring, so as to maintain the temperature at or below 15° C.

Stirring was then continued for 30 minutes at 15° to 20° C.

At the end, the reaction mixture was poured into 30 ml of aqueous $(NH_4)_2SO_4$ (20%), maintained under stirring at 5° C, and the resulting solution was extracted with $CH_2Cl_2$ (8 x 30 ml). The organic extract was washed with 30 ml of $(NH_4)_2SO_4$ (40%), dried over anhydrous $Na_2SO_4$, filtered and evaporated at 30° C under vacuum.

Obtained were 1.1 g of crystalline, essentially pure N-acetyl4-piperidine percarboxylic acid.
Yield: 50%.

Elemental Analysis:

| | |
|---|---|
| Calculated for $C_8H_{13}O_4N$ : | C = 51.33%; H = 7,0%; N = 7.48%; O (active) = 8.54%. |
| Found: | C = 51.10%; H = 6.97%; N = 7.39%; O (active) = 8.53%. |

Melting point: 98% (with decomposition).

## EXAMPLE 2

3 g of N-decanoyl-4-piperidine carboxylic acid (0.0106 moles) were completely dissolved, at 25° C, in 14 g of methanesulphonic acid (0.146 moles).

By following the procedure of example 1, 2 g of $H_2O_2$ (85%, 0.05 moles) were then added in a way such as to not exceed 15° C. Stirring was then continued at a temperature of 15 to 20° C for 1.5 hours. At the end, the reaction mixture was poured into 40 ml of $(NH_4)_2SO_4$ (20%) maintained under stirring at 5° C and was further worked up according to example 1 by extracting the solution with $CH_2Cl_2$ (3 x 40 ml). After

evaporation of the organic extract, 2.8 g of crystalline, essentially pure N-decanoyl-4-piperidine percarboxylic acid were obtained.
Yield: 88%.

Elemental Analysis:

| | |
|---|---|
| Calculated for $C_{16}H_{29}O_4N$: | C = 64.18%; H = 9.76%; N = 4.76%; O (active) = 5.34%. |
| Found: | C = 64.23%; H = 9.89%; N = 4.66%; O (active) = 5.33%. |

Melting point: 52°C (with decomposition).

## EXAMPLE 3

6 g of N, N′-adipoyl-bis (4-piperidine carboxylic acid) (0.0163 moles) were suspended, under stirring, in 42 g of methansulphonic acid (0.437 moles).
6 g of $H_2O_2$ (85%, 0.15 moles) were added to the resulting cloudy solution, maintained at 5°C, so as to not exceed 15°C. Stirring was then continued at a temperature of 15 to 20°C for 2.5 hours.

At the end, the reaction mixture was poured into 150 ml of aqueous $(NH_4)_2SO_4$ (10%) maintained under stirring at 5°C. After 30 minutes, the solid product was filtered over a porous septum under vacuum and consecutively washed with ice water (3 x 30 ml), tetrahydrofuran (30 ml) and ethyl ether (2 x 30 ml). Then the product was dried under vacuum in a $CaCl_2$ drier for 2 hours at room temperature. Obtained were 6.1 g of N, N′-adipoyl-bis (4-piperidine percarboxylic acid).
Yield: 94%.

Elemental Analysis:

| | |
|---|---|
| Calculated for $C_{18}H_{28}O_8N_2$: | C = 53.99%; H = 7.05%; N = 6.99%; O (active) = 7.99%. |
| Found: | C = 53.32%; H = 7.19%; N = 6.64%; O (active) = 7.98%. |

Melting point: 141°C (with decompsition).

## EXAMPLE 4

4.5 g of N-decanoyl-3-piperidine carboxylic acid (0.0158 moles) were completely dissolved in 21 g of methanesulphonic acid (0.218 moles).
Following the procedure of example 1, 3 g of $H_2O_2$ (85%, 0.075 moles) were added to this mixture so that the temperature was maintained at or below 15°C. Stirring was then continued at a temperature of 15 to 20°C for 1.5 hours. At the end, the reaction mixture was poured into 50 ml of $(NH_4)_2SO_4$ (20%) maintained under stirring at 5°C, and was further worked up as described in example 1 by extracting the resulting solution with $CH_2C_{12}$ (3 x 50 ml).
After the evaporation of the solvent, 3.9 g of crystalline, N-decanoyl-3-piperidine percarboxylic acid (purity 99%) were obtained.
Yield: 80%.

Elemental Analysis:

| | |
|---|---|
| Calculated for $C_{16}H_{29}O_4N$: | C = 64.18%; H = 9.76%; N = 4.67%; O (active) = 5.34%. |
| Found: | C = 64.18%; H = 9.84%; N = 4.65%; O (active) = 5.28%. |

Melting point: 48° C (with decomposition).

EXAMPLE 5

By following the procedure of example 1, 4.3 g of $H_2O_2$ (85%, 0.107 moles) were added to a mixture of 4 g of N, N′-succinyl-bis (3-piperidine carboxylic acid) (0.018 moles) and 29 g of methanesulphonic acid, so that the temperature was maintained at or below 15° C. Stirring was then continued at a temperature of 15 to 20° C for 2.5 hours. At the end, the reaction mixture was poured into 100 ml of $(NH_4)_2SO_4$ (20%), maintained under stirring at 5° C.

After 30 minutes, the formed solid product was filtered and then the procedure of example 3 was followed. Obtained were 3.8 g of crystalline, essentially pure N, N′-succinyl-bis (3-piperidine percarboxylic acid).
Yield: 86%.

Elemental Analysis:

| | |
|---|---|
| Calculated for $C_{16}H_{24}O_8N_2$: | C = 51.6%; H = 6.49%; N = 7.25%; O (active) = 8.59%. |
| Found: | C = 51.42%; H = 6.52%; N = 7.31%; O (aktiv) = 8.58%. |

The product decomposes at about 130° C without melting.

EXAMPLE 6 (Application Example)

Bleaching tests were carried out with the novel compounds listed in the annexed Tables 1 and 2, at alkaline pH (Table 1) and acid pH (Table 2). The following substance served as comparison:
H 48 (Mg salt of monoperphthalic acid), a commercial peracid known in the detergent art, and manufactured by INTEROX Chemical Ltd., London. U.K. (Tables 1 and 2).

All tests were carried out at constant temperature (60° C), with an initial concentration of total active oxygen in the bleaching solution (identical for all products) of equal to 200 mg/l.

Process

For each test, 500 ml of deionized water, contained in a 1000 ml flask equipped with condenser, was heated to a temperature of 60° C and adjusted to a pH of 9.5 (by adding a few drops of an NaOH solution) (Table 1) and of 2 - 3 (by adding a few drops of diluted $H_2SO_4$) (Table 2). Then the bleaching product was added with stirring, in amounts shown in the following Tables, and immediately thereafter two cotton specimens of 10 cm x 10 cm, stained with standard stains of red wine at EMPA INSTITUTE of St.Gallen (Switzerland), and marked with the "EMPA 114" mark, were added.

The system subsequently was kept under stirring for 60 minutes and, at the end of this time, the specimens, rinsed under running water, were dried and ironed, and were then subjected to an evaluation of the bleaching effect by means of a measurement of the whiteness degree of reflectometry. The results are reported in the following Tables 1 and 2, wherein the data are expressed as % Bleaching, defined as:

$$\% \text{ Bleaching} = \frac{A - B}{C - B} \times 100\%$$

wherein:
A = degree of whiteness (%) of the specimen bleached after the test;

B = degree of whiteness (%) of the specimen before the test;
C = degree of whiteness (%) of the completely bleached specimen.

The degree of whiteness was measured by means of an Elrepho Zeiss reflectometer, assuming MgO = 100% of whiteness, and using a filter N.6 (λ = 464 nm).

The data listed in Table 1, concerning tests carried out at alkaline pH, show that the peracids of the present invention have a bleaching power which may be compared to the bleaching power of H 48.

Likewise, the results, expressed as % Bleaching, listed in Table 2, show that the tested products have a bleaching power in acid solution which is particularly high and higher than that of H 48.

This is particularly surprising in consideration of the fact that the peroxidic compounds generally show a very modest and sometimes negligible bleaching activity at acid pH.

TABLE 1

| Tests carried out at alkaline pH (9.5) | | | |
|---|---|---|---|
| Compound | Amounts used in the tests (grams) | Initial concentration of total active oxygen (mg/l) | % Bleaching |
| - Example 2 (titer = 5.33% of active oxygen) | 1.88 | 200 | 75.4 |
| - Example 3 (titer = 7.98% of active oxygen) | 1.25 | 200 | 81.8 |
| - H 48 (titer = 5.5% of active oxygen) | 1.86 | 200 | 80.0 |

TABLE 2

| Tests carried out at acid pH (2-3) | | | |
|---|---|---|---|
| Compound | Amounts used in the tests (grams) | Initial concentration of total active oxygen (mg/l) | % Bleaching |
| - Example 2 (titer = 5.33% of active oxygen) | 1.88 | 200 | 70.3 |
| - H 48 (titer = 5.5% of active oxygen) | 1.86 | 200 | 60.0 |

## Claims

1. Heterocyclic (poly)percarboxylic acids of formula:

$R-(CH_2)_m-R'$ (I)

wherein

R and R', the same or different from each other, represent hydrogen or a group of the following formula:

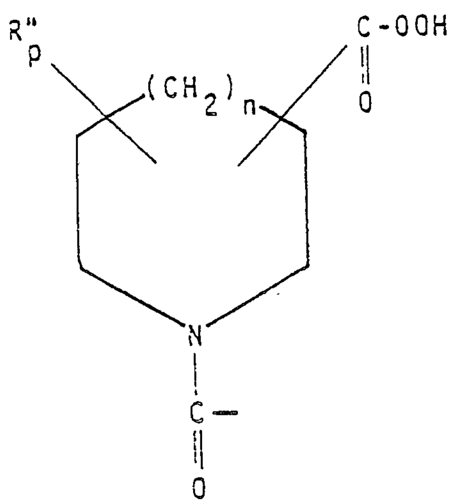

provided that at least one of R and R′ is different from H, and wherein:
R″ represents hydrogen or any other substituent non-reactive in the presence of the active oxygen of the percarboxylic acid group and/or under the preparation conditions; m represents a number of from 1 to 12;
n is selected from 0,1 and 2; and
p represents a number of from 1 to 3.

2. Heterocyclic (poly)percarboxylic acids according to claim 1, wherein R″ is an alkyl, cycloalkyl, alkyl-aryl or aryl-alkyl group containing an overall number of up to 10 carbon atoms and being optionally substituted by at least one substituent selected from F, Cl, $NO_2$ and lower ($C_1$-$C_5$)-alkoxy groups, or represents a carboxylic group, F, Cl or ($C_1$-$C_5$)alkoxy.

3. Heterocyclic (poly)percarboxylic acids according to anyone of claims 1 and 2, wherein n is equal to 1 and R is a hydrogen atom.

4. Compounds according to claim 1, i.e. N-acetyl-4-piperidine percarboxylic acid, N-decanoyl-4-piperidine percarboxylic acid, N, N′-adipoyl-bis (4-piperidine percarboxylic acid), N-decanoyl-3-piperidine percarboxylic acid, or N, N′-succinyl-bis (3-piperidine percarboxylic acid).

5. Process for preparing the (poly)percarboxylic acids of formula (I), characterized in that a (poly)-carboxylic acid, corresponding in structure to the desired percarboxylic acid of formula (I), is reacted with concentrated $H_2O_2$ by operating in an acid medium, preferably methanesulphonic acid, whereafter the resulting percarboxylic acid of formula (I) is separated from the reaction mixture according to conventional methods.

6. Use of the heterocyclic (poly)percarboxylic acids according to anyone of claims 1 to 4 in detergent formulations.

7. Use according to claim 6, characterized in that said formulations contain other components and/or substances selected from additives, builder, surfactants, soaps, zeolites, hydrotropic agents, corrosion inhibitors, enzymes, optical bleaching agents, stabilizers and other peroxidic compounds.

European Patent Office

# EUROPEAN SEARCH REPORT

Application Number

EP 89 11 0722

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 260 134 (THE PROCTER & GAMBLE COMPANY)<br>* page 9, claim 1 * | 1 | C 07 D 211/46<br>C 07 D 207/16<br>C 07 D 223/08<br>C 11 D 3/395 |
| Y | EP-A-0 170 386 (THE PROCTER & GAMBLE COMPANY)<br>* page 1, claim 1; page 23, example VIII * | 1 | |
| X | | 5 | |
| Y | EP-A-0 233 476 (PENNWALT CORP.)<br>* page 15, claim 1 * | 1 | |

TECHNICAL FIELDS SEARCHED (Int. Cl.4)

C 07 D 207/00
C 07 D 211/00
C 07 D 223/00
C 11 D 3/00

The present search report has been drawn up for all claims

| Place of search | Date of completion of the search | Examiner |
|---|---|---|
| BERLIN | 21-09-1989 | KYRIAKAKOU G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)